(19) Europäisches Patentamt
European Patent Office
Office européen des brevets

(11) EP 1 238 647 A1

(12) **DEMANDE DE BREVET EUROPEEN**

(43) Date de publication:
**11.09.2002 Bulletin 2002/37**

(51) Int Cl.7: **A61K 7/06**, A61K 7/48

(21) Numéro de dépôt: **02290453.6**

(22) Date de dépôt: **25.02.2002**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **05.03.2001 FR 0102980**

(71) Demandeur: **L'OREAL**
**75008 Paris (FR)**

(72) Inventeurs:
- **Philippe, Michel**
  **91320 Wissous (FR)**
- **Benard, Sylvie**
  **95570 Attainville (FR)**
- **Blaise, Christian**
  **94160 Saint-Mande (FR)**

(74) Mandataire: **Dodin, Catherine**
**L'OREAL-DPI**
**6 rue Bertrand Sincholle**
**92585 Clichy Cédex (FR)**

(54) **Utilisations de dérivés de polyaminoacide comme agent hydratant, et compositions cosmétique ou pharmaceutique les comprenant**

(57) L'invention a pour objet l'utilisation de dérivés de polyaminoacide pour la préparation d'une composition destinée à hydrater la peau, les muqueuses et/ou les fibres kératiniques, et/ou comme agent hydratant et/ou émollient, notamment de la peau, des muqueuses et/ou des fibres kératiniques.

L'invention concerne également une composition cosmétique ou pharmaceutique destinée à améliorer l'hydratation de la peau, des muqueuses et/ou des fibres kératiniques, comprenant au moins un tel dérivé de polyaminoacide.

EP 1 238 647 A1

## Description

La présente invention a pour objet l'utilisation de dérivés de polyaminoacides dans une composition notamment cosmétique destinée à être appliquée sur la peau ou les muqueuses, afin d'en améliorer l'hydratation ou d'en prévenir le dessèchement.

On connaît différentes classes de polyaminoacides décrites dans la littérature, pur leurs nombreuses utilisations notamment en cosmétique.

Ainsi, la demande japonaise JP-07/041,467 décrit une classe de polyaminoacides de poids moléculaire élevé constitués essentiellement de cystéine.

La demande japonaise JP-06/248,072 décrit une classe de polyaminoacides caractérisée par la présence de fonctions thiols et/ou disulfures; ces polyaminoacides réagissent avec les liaisons thiols de la kératine formant ainsi des ponts disulfures, ce qui permet de rehausser les qualités de brillance et de coloration de la chevelure.

La demande française FR-2533209 décrit des lipopeptides amphipatiques constitués d'une chaîne peptidique hydrophile et d'une chaîne hydrophobe de 8 à 24 atomes de carbone, ainsi que leur utilisation comme agents émulsifiants de milieux non miscibles ou pour l'obtention de cristaux liquides.

La demande FR2776510 décrit des compositions cosmétiques destinées au renforcement ou au soin des fibres kératiniques, notamment capillaires, qui comprennent des dérivés de polyaminoacides.

Des polyaminoacides constitués essentiellement d'acides aminés à chaînes neutres et acides ont été décrits dans la demande japonaise JP-04/198,114, et sont utilisés de manière générale en tant qu'agent hydratant. Les composés décrits dans cette demande sont des copolymères d'aminoacide à chaîne acide avec un aminoacide à chaîne neutre. La copolymérisation nécessite, au préalable, une activation de chacun des aminoacides; par ailleurs, il est également nécessaire de protéger la fonction acide de l'aminoacide à chaîne acide avant l'étape d'activation. Ces deux étapes de protection et d'activation rendent le procédé de synthèse particulièrement complexe, et peu intéressant économiquement et industriellement.

La présente invention a pour but de pallier les inconvénients de l'art antérieur et de proposer une nouvelle classe de dérivés de polyaminoacides, susceptibles d'être obtenus par un procédé simple et industriel, et qui présentent de bonnes propriétés d'hydratation.

L'invention a donc pour objet l'utilisation d'au moins un dérivé de polyaminoacide de formule (I) telle que définie ci-après, pour la préparation d'une composition destinée à hydrater la peau, les muqueuses et/ou les fibres kératiniques, et/ou d'une composition destinée à être appliquée sur la peau, les muqueuses et/ou les fibres kératiniques afin d'en améliorer l'hydratation ou d'en prévenir le dessèchement.

L'invention a également pour objet l'utilisation d'au moins un dérivé de polyaminoacide de formule (I) telle que définie ci-après, comme agent hydratant et/ou émollient, notamment de la peau, des muqueuses et/ou des fibres kératiniques.

Un autre objet de l'invention est une composition cosmétique ou pharmaceutique destinée à améliorer l'hydratation de la peau, des muqueuses et/ou des fibres kératiniques, comprenant au moins un dérivé de formule (I) tel que défini ci-après.

On a constaté que les composés selon l'invention présentent un très bon pouvoir hydratant de la peau, des muqueuses et des fibres kératiniques (cheveux, cils, sourcils et ongles). Ils peuvent être utilisés avec un intérêt tout particulier lorsqu'un effet de lutte contre le dessèchement de la peau et/ou des cheveux est recherché en cosmétique ou en pharmaceutique.

Ces composés ont l'avantage d'améliorer et/ou de rétablir la fonction barrière lorsqu'ils sont appliqués sur la peau.

Ils présentent des propriétés émollientes et adoucissantes, notamment de la peau et des cheveux. Ils peuvent en outre être facilement solubilisés dans les phases grasses des compositions cosmétiques ou pharmaceutiques.

Les composés selon l'invention peuvent être utilisés pour traiter les peaux abîmées et/ou âgées, ainsi que les cheveux ou les ongles abîmés, dans le domaine cosmétique ou pharmaceutique.

Par ailleurs, la synthèse de ces composé est très rapide, et aisément industrialisable.

Les composés employés dans la présente invention sont donc des homopolymères répondant à la formule (I):

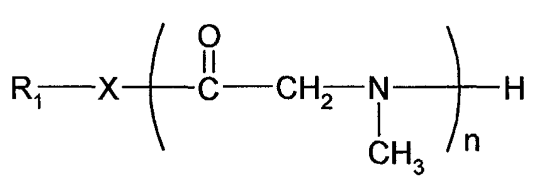

(I)

dans laquelle :

- X est choisi parmi -O-, -S- ou -NR, avec R représentant un atome d'hydrogène ou un radical hydrocarboné, linéaire ou ramifié, saturé ou insaturé en $C_1$-$C_6$,

- $R_1$ est choisi parmi :

  (i) un atome d'hydrogène,
  (ii) un radical hydrocarboné, linéaire ou ramifié, saturé ou insaturé, en $C_1$-$C_{40}$, éventuellement substitué par au moins un hydroxy ou un radical -NR'R", dans lequel R' et R" représentent, indépendamment l'un de l'autre, un atome d'hydrogène ou un radical hydrocarboné, linéaire ou ramifié, saturé ou insaturé en $C_1$-$C_6$; et/ou éventuellement interrompu par au moins un hétéroatome choisi parmi N, O ou Si,
  (iii) un radical choisi parmi

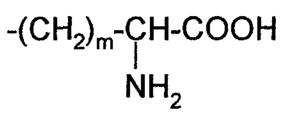 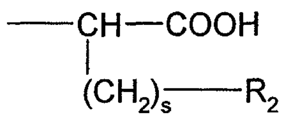 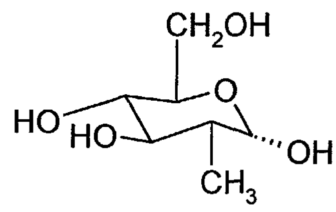

  dans lesquels :

  - m est 1, 2, 3, 4 ou 5;
  - s est un entier compris entre 0 et 4 inclus;
  - R2 représente un atome d'hydrogène, $-NH_2$, -OH, -SH, $-CHOHCH_3$, $-CONH_2$,

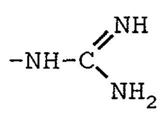 , 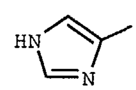 ,

    $-C_6H_5$ ou $-C_6H_5pOH$,
  - n est un nombre moyen d'unités répétitives supérieur à 1, et tel que le poids moléculaire du dérivé de polyaminoacide est compris entre 200 et 200.000.

Les composés de formule (I) peuvent également se présenter sous forme de sels minéraux ou organiques, compatibles avec une application dans les domaines cosmétique ou pharmaceutique.
De préférence, X représente -NR avec R représentant un atome d'hydrogène ou un radical hydrocarboné, linéaire ou ramifié, saturé, en $C_1$-$C_4$, notamment un radical méthyle ou éthyle.
De préférence, $R_1$ représente :

- un radical hydrocarboné, linéaire ou ramifié, saturé ou insaturé, en $C_1$-$C_{22}$, notamment en C4-20, éventuellement substitué par au moins un hydroxy, voire par deux, trois, quatre ou cinq -OH, ou
- un radical choisi parmi :

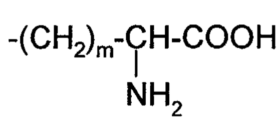 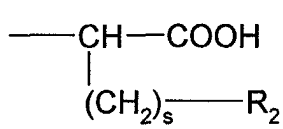 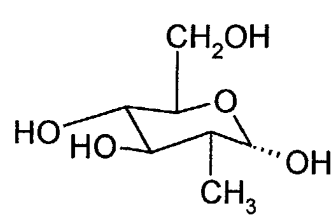

dans lesquels :

- m est 1,2, 3, 4 ou 5;
- s est un entier compris entre 1 et 3 inclus;
- R2 représente $-CONH_2$,

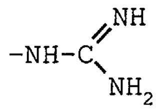 , 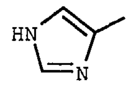 .

En particulier, on peut citer les composés de formule (I) dans lesquels le radical $R_1$ représente l'une des formules suivantes :

$$C_{15}H_{31}-CH(OH)-CH(CH_2OH)-$$

$$C_{10}H_{21}-CH(C_8H_{17})-CH_2-$$

$$C_{16}H_{33}-$$

$$C_8H_{17}-CH=CH-C_8H_{16}-$$

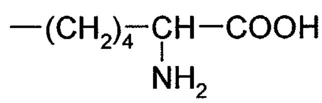

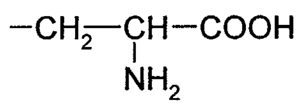

$R_1$ = 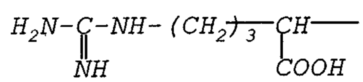

$R_1$ = 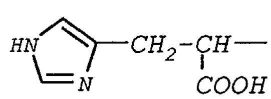 ,

$R_1$ = 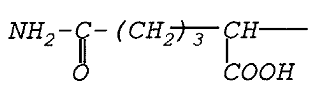 ,

$$CH_2(OH)-CH(OH)-CH(OH)-CH(OH)-CH(OH)-CH_2-$$

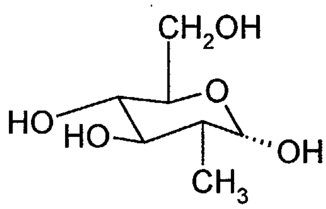

De préférence, n est compris entre 3 et 500 et/ou est tel que le poids moléculaire du dérivé de polyaminoacide est compris entre 300 et 50 000.

Les dérivés de polyaminoacides de formule (I) peuvent être obtenus par des procédés bien connus de l'homme du métier, et notamment grâce à une réaction de polycondensation entre au moins un N-carboxyanhydride de formule :

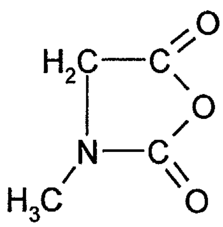

et un composé nucléophile de formule $R_1$-XH dans laquelle $R_1$ et X ont les mêmes significations que celles données ci-dessus pour la formule (I).

Ce procédé est notamment décrit dans la demande française FR2776510.

Les dérivés de polyaminoacide peuvent être utilisés, seuls ou en mélange, en une quantité de 0,001 à 30% en poids par rapport au poids total de la composition, de préférence en une quantité de 0,01 à 15% en poids par rapport au poids total de la composition.

Les composés selon l'invention peuvent recevoir des applications diverses, notamment dans des compositions cosmétiques ou pharmaceutiques, qui comprennent alors un milieu physiologiquement acceptable, notamment cosmétiquement ou pharmaceutiquement acceptable.

Ce milieu, ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux.

Lorsque la composition comprend une phase aqueuse, ladite phase peut comprendre de l'eau, une eau florale et/ou une eau minérale.

Ladite phase peut comprendre en outre des alcools tels que des monoalcools en $C_1$-$C_6$ et/ou des polyols tels que le glycérol, le butylèneglycol, l'isoprène glycol, le propylèneglycol, le polyéthylèneglycol.

La composition peut également comprendre une phase grasse, notamment constituée de corps gras liquides à 25°C, tels que des huiles d'origine animale, végétale, minérale ou synthétique, volatiles ou non; de corps gras solides à 25°C tels que des cires d'origine animale, végétale, minérale ou synthétique; de corps gras pâteux; de gommes; de leurs mélanges.

Les huiles volatiles sont généralement des huiles ayant, à 25°C, une tension de vapeur saturante au moins égale à 0,5 millibar (soit 50 Pa).

Parmi les constituants de la phase grasse, on peut citer :

- les silicones volatiles cycliques ayant de 3 à 8 atomes de silicium, de préférence de 4 à 6;
- les cyclocopolymères du type diméthylsiloxane/méthylalkylsiloxane,
- les silicones volatiles linéaires ayant de 2 à 9 atomes de silicium;
- les huiles volatiles hydrocarbonées, telles que les isoparaffines et notamment l'isododécane et des huiles fluorées;
- les polyalkyl($C_1$-$C_{20}$) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des

groupements fonctionnels tels que des groupements hydroxyles, thiols et/ou amines.

- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule $R1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.
- les gommes de silicones;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

La composition peut comprendre en outre tout additif usuellement utilisé dans le domaine d'application envisagé, tel que des tensioactifs, des antioxydants, des parfums, des huiles essentielles, des conservateurs, des actifs cosmétiques ou pharmaceutiques, des vitamines, des acides gras essentiels, des sphingolipides, des agents autobronzants, des filtres solaires, des polymères filmogènes, des épaississants, des colorants, des pigments, des charges, des nacres.
Bien entendu l'homme du métier veillera à choisir les éventuels composés complémentaires et leur quantité, de manière telle que les propriétés avantageuses de la composition selon l'invention ne soient pas, ou substantiellement pas, altérées par l'adjonction envisagée.

Les compositions selon l'invention sont destinées à être appliquées sur la peau du visage et du corps, sur les muqueuses et/ou sur les fibres kératiniques telles que les ongles, les cils ou les cheveux.
Elles peuvent se présenter sous toutes les formes galéniques envisageables, telles que solution huileuse ou aqueuse; gel huileux ou aqueux; émulsion huile-dans-eau, eau-dans-huile ou multiple; dispersion huile dans l'eau ou eau dans huile; système multiphases notamment biphase.

Ces compositions peuvent se présenter :

- sous la forme d'un produit de maquillage de la peau du visage, du corps ou des lèvres ou des fibres kératiniques (ongles, cils, sourcils, cheveux), tel qu'un fond de teint, une crème teintée, un fard à joues ou à paupières, une poudre libre ou compacte, un stick anti-cernes, un stick camouflant, un mascara, un rouge à lèvres, un vernis-à-ongles,
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil;
- sous la forme d'une composition capillaire, et notamment une crème ou un gel de soin des cheveux, notamment un produit hydratant,
- d'une composition de protection solaire, de bronzage artificiel (autobronzant) ou de soin après-soleil.

L'invention est illustrée plus en détail dans les exemples suivants.

## Exemple 1

Préparation du composé de formule (I) dans laquelle :
$R_1 = CH_2(OH)\text{-}CH(OH)\text{-}CH(OH)\text{-}CH(OH)\text{-}CH(OH)\text{-}CH_2\text{-}$, X = -NH- et n=12 (indice théorique)

Dans un erlenmeyer de 500 ml, sous agitation, on introduit 10 g (0,08 mole) de N-carboxyanhydride sarcosine, puis on ajoute 100 ml d'eau distillée (pH 6,7) et en une seule fois 0,006 mole de glucamine.
Un dégagement important de $CO_2$ se produit, puis on poursuit l'agitation du mélange pendant environ 30 minutes à 25°C.

Après évaporation de l'eau sous pression réduite et séchage sous vide, on obtient une poudre de couleur beige pâle.

**Exemple 2**

Préparation du composé formule (I) dans laquelle :
$R_1 = CH_2(OH)\text{-}CH(OH)\text{-}CH(OH)\text{-}CH(OH)\text{-}CH(OH)\text{-}CH_2\text{-}$, X = $-NCH_3-$ et n=12 (indice théorique)
On prépare ce composé selon le même mode opératoire que celui décrit à l'exemple 1, en remplaçant la glucamine par la quantité molaire correspondante de N-méthylglucamine.

**Exemple 3**

Préparation du composé formule (I) dans laquelle:

R1 =

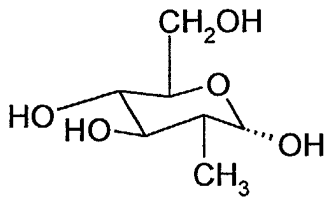

X = -NH- et n=12 (indice théorique)
On prépare ce composé selon le même mode opératoire que celui décrit à l'exemple 1, en remplaçant la glucamine par la quantité molaire correspondante de glucosamine préalablement désalifiée.

**Exemple 4: crème de jour**

On prépare une crème de soin du visage, hydratante, comprenant :

- cétyl alcool 2,5 g
- tristéarate de sorbitan 0,9 g
- stéarate de PEG 40 2 g
- stéarate de glycéryle 3 g
- myristate de myristyle 2 g
- polyisobutène hydrogéné 2,5 g
- palmitate d'octyle 4 g
- polydiméthylsiloxane (10 $cm^2.s$) 5 g
- huile de noyaux d'abricot 5,7 g
- composé de l'exemple 2 0,5 g
- conservateurs, parfum qs
- eau qsp 100 g

On obtient une crème de jour se présentant sous la forme d'une émulsion permettant de bien couvrir et de protéger la peau; cette crème est particulièrement adaptée pour les peaux normales et sèches.

**Exemple 5: crème de nuit**

On prépare une crème de soin du visage, hydratante, comprenant :

- mélange de mono- et di-stéarate de glycéryle et de stéarate de POE 2 g
- huile de noyaux d'abricot 17 g
- cyclopentadiméthylsiloxane 1,5 g
- carbomer 0,75 g
- triéthanolamine 0,75 g
- composé de l'exemple 1 3 g
- conservateurs qs
- eau qsp 100 g

On obtient une crème de nuit se présentant sous la forme d'une émulsion épaissie, brillante et douce à appliquer. La crème est nourrissante et hydratante pour la peau, et est particulièrement adaptée pour les peaux sèches.

**Exemple 6 : mesure de l'hydratation**

On a mesuré in vitro l'hydratation apportée par 3 composés selon l'invention par mesure du module d'élasticité du stratum corneum, avec un appareil de type Dermomètre. Cet appareil a été décrit par L. Rasseneur et al. dans Influence des Différents Constituants de la Couche Cornée sur la Mesure de son Elasticité, International Journal of Cosmetic Science, 4, 247-260 (1982).
Le principe consiste à mesurer avant traitement le module d'élasticité du stratum corneum au début du palier de fluage, caractéristique des matériaux viscoélastiques. Le module d'élasticité après traitement est alors déterminé à la même élongation du stratum corneum .
On sait par ailleurs que le module d'élasticité diminue fortement quand la teneur en eau, donc l'hydratation, augmente.
L'indice de diminution du module caractérise l'effet hydratant du traitement :

$$100 \times (E_{traité} - E_{témoin}) / E_{témoin}$$

Ce test a été réalisé in vitro sur du stratum corneum isolé sur lequel on a appliquer une solution à 3% en poids dans l'eau du composé à tester.
On utilise des éprouvettes de 0,6 x 0,4 cm de stratum corneum, d'épaisseur comprise entre 10 et 20 microns, disposées entre les mors du Dermomètre.
L'éprouvette est placée entre 2 mâchoires puis laissée pendant 12 heures dans une atmosphère à 30°C et 75% d'humidité relative. Ces mâchoires sont alors fixées sur le Dermomètre.
On tracte à la vitesse de 1 mm/minute l'éprouvette d'une longueur comprise entre 5 et 10 % de la longueur initiale pour déterminer la longueur $L_0$ à partir de laquelle l'éprouvette commence à exercer une force sur les mâchoires et détectée par l'appareil.
On détend ensuite l'éprouvette puis on applique sur le stratum cornéum 2 mg de la solution à tester. Après évaporation totale, on tracte l'éprouvette dans les mêmes conditions que celles décrites précédemment pour déterminer également la longueur $L_1$ pour l'éprouvette traitée.
Le pourcentage de rétraction est déterminé par le rapport : $100 \times (L_1 - L_o)/L_o$
Pour caractériser un effet tenseur, ce pourcentage doit être négatif et l'effet tenseur est d'autant plus important que la valeur absolue du pourcentage de rétraction est élevée.
Des mesures sont effectuées aux temps $T_0$, $T_1$, $T_2$ et $T_4$, c'est-à-dire juste avant le traitement, et 2 heures, 4 heures, 6 heures et 20 heures après l'application du composé à tester.
On obtient les résultats suivants (moyenne sur 5 échantillons) :

| Variation du module d'élasticité de l'échantillon de stratum corneum en % : | | | | |
|---|---|---|---|---|
| | Après 2 heures | Après 4 heures | Après 6 heures | Après 20 heures |
| Solution aqueuse à 3% du composé de l'exemple 1 | -21% | -29% | -34% | -38% |
| Solution aqueuse à 3% du composé de l'exemple 2 | -29% | -38% | -45% | -49% |
| Solution aqueuse à 3% du composé de l'exemple 3 | -31% | -32% | -28% | -42% |
| Eau | +1% | -1% | 0 | -2% |

Ces résultats montrent que les composés selon l'invention possèdent une forte activité hydratante avec une singularité très importante au niveau de l'expression de l'activité, puisque cette dernière augmente dans le temps (augmentation entre les résultats à 2 heures et à 20 heures).

## Revendications

1. Utilisation d'au moins un dérivé de polyaminoacide de formule (I)

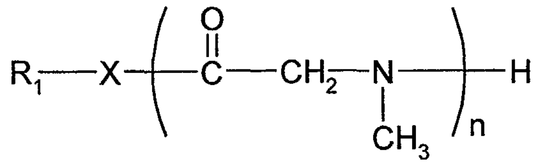 (I)

dans laquelle :

- X est choisi parmi -O-, -S- ou -NR, avec R représentant un atome d'hydrogène ou un radical hydrocarboné, linéaire ou ramifié, saturé ou insaturé en $C_1$-$C_6$,

- $R_1$ est choisi parmi :

  (i) un atome d'hydrogène,
  (ii) un radical hydrocarboné, linéaire ou ramifié, saturé ou insaturé, en $C_1$-$C_{40}$, éventuellement substitué par au moins un hydroxy ou un radical -NR'R", dans lequel R' et R" représentent, indépendamment l'un de l'autre, un atome d'hydrogène ou un radical hydrocarboné, linéaire ou ramifié, saturé ou insaturé en $C_1$-$C_6$; et/ou éventuellement interrompu par au moins un hétéroatome choisi parmi N, O ou Si,
  (iii) un radical choisi parmi

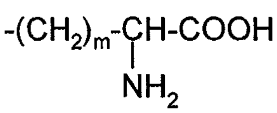 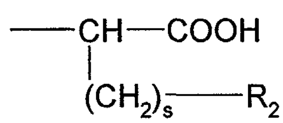 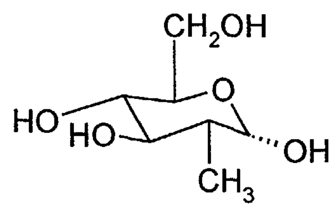

  dans lesquels :

  - m est 1, 2, 3, 4 ou 5;
  - s est un entier compris entre 0 et 4 inclus;
  - R2 représente un atome d'hydrogène, $-NH_2$, -OH, -SH, $-CHOHCH_3$, $-CONH_2$,

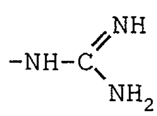 , 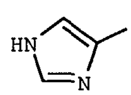 ,

    $-C_6H_5$ ou $-C_6H_5pOH$,
  - n est un nombre moyen d'unités répétitives supérieur à 1, et tel que le poids moléculaire du dérivé de polyaminoacide est compris entre 200 et 200.000,

ou d'un de ses sels,
pour la préparation d'une composition destinée à hydrater la peau, les muqueuses et/ou les fibres kératiniques, et/ou d'une composition destinée à être appliquée sur la peau, les muqueuses et/ou les fibres kératiniques afin d'en améliorer l'hydratation ou d'en prévenir le dessèchement.

**2.** Utilisation d'au moins un dérivé de polyaminoacide de formule (I)

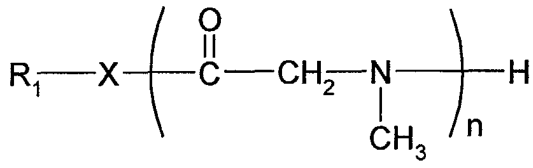 (I)

dans laquelle :

- X est choisi parmi -O-, -S- ou -NR, avec R représentant un atome d'hydrogène ou un radical hydrocarboné, linéaire ou ramifié, saturé ou insaturé en $C_1$-$C_6$,

- $R_1$ est choisi parmi :

  (i) un atome d'hydrogène,
  (ii) un radical hydrocarboné, linéaire ou ramifié, saturé ou insaturé, en $C_1$-$C_{40}$, éventuellement substitué par au moins un hydroxy ou un radical -NR'R", dans lequel R' et R" représentent, indépendamment l'un de l'autre, un atome d'hydrogène ou un radical hydrocarboné, linéaire ou ramifié, saturé ou insaturé en $C_1$-$C_6$; et/ou éventuellement interrompu par au moins un hétéroatome choisi parmi N, O ou Si,
  (iii) un radical choisi parmi

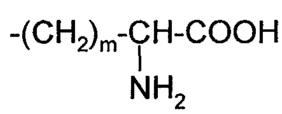

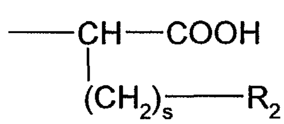

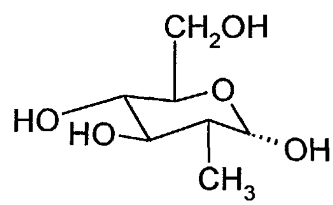

  dans lesquels :

  - m est 1, 2, 3, 4 ou 5;
  - s est un entier compris entre 0 et 4 inclus;
  - R2 représente un atome d'hydrogène, $-NH_2$, -OH, -SH, $-CHOHCH_3$, $-CONH_2$,

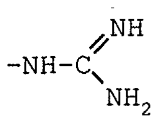 , 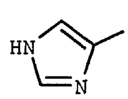 ,

    $-C_6H_5$ ou $-C_6H_5pOH$,
  - n est un nombre moyen d'unités répétitives supérieur à 1, et tel que le poids moléculaire du dérivé de polyaminoacide est compris entre 200 et 200.000,

ou d'un de ses sels,
comme agent hydratant et/ou émollient, notamment de la peau, des muqueuses et/ou des fibres kératiniques.

**3.** Utilisation selon l'une des revendications précédentes, dans laquelle X représente -NR avec R représentant un atome d'hydrogène ou un radical hydrocarboné, linéaire ou ramifié, saturé, en $C_1$-$C_4$, notamment un radical méthyle ou éthyle.

**4.** Utilisation selon l'une des revendications précédentes, dans laquelle $R_1$ représente :

- un radical hydrocarboné, linéaire ou ramifié, saturé ou insaturé, en $C_1$-$C_{22}$, notamment en C4-20, éventuellement substitué par au moins un hydroxy, voire par deux, trois, quatre ou cinq -OH, ou
- un radical choisi parmi:

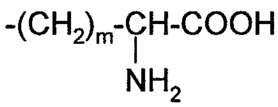 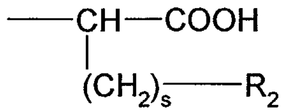 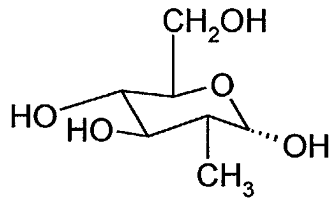

dans lesquels :

- m est 1, 2, 3, 4 ou 5;
- s est un entier comoris entre 1 et 3 inclus:
- R2 représente $-CONH_2$,

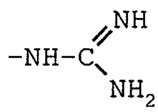 , 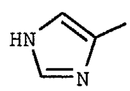 .

**5.** Utilisation selon l'une des revendications précédentes, dans laquelle le radical $R_1$ représente l'une des formules suivantes :

$C_{15}H_{31}$-CH(OH)-CH($CH_2$OH)-

$C_{10}H_{21}$-CH($C_8H_{17}$)-$CH_2$-

$C_{16}H_{33}$-

$C_8H_{17}$-CH=CH-$C_8H_{16}$-

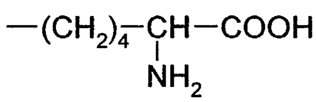

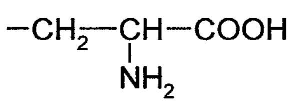

$R_1$ = 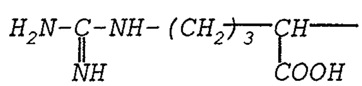

$R_1$ = 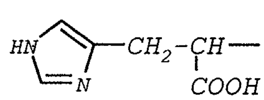 ,

$R_1$ = 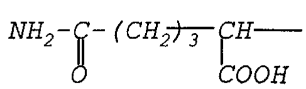 ,

$$CH_2(OH)-CH(OH)-CH(OH)-CH(OH)-CH(OH)-CH_2-$$

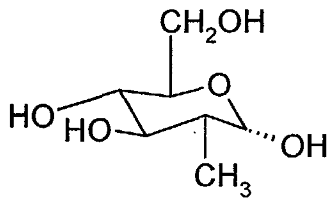

6. Utilisation selon l'une des revendications précédentes, dans laquelle n est compris entre 3 et 500 et/ou est tel que le poids moléculaire du dérivé de polyaminoacide est compris entre 300 et 50 000.

7. Utilisation selon l'une des revendications précédentes, dans laquelle les dérivés de polyaminoacide sont utilisés, seuls ou en mélange, en une quantité de 0,001 à 30% en poids par rapport au poids total de la composition, de préférence en une quantité de 0,01 à 15% en poids par rapport au poids total de la composition.

8. Utilisation selon l'une des revendications précédentes, dans des compositions cosmétiques ou pharmaceutiques, comprenant par ailleurs un milieu physiologiquement acceptable, notamment cosmétiquement ou pharmaceutiquement acceptable.

9. Utilisation selon l'une des revendications précédentes, dans un produit de maquillage de la peau du visage, du corps ou des lèvres ou des fibres kératiniques ; dans un produit de soin, de traitement, de nettoyage ou de protection, de la peau du visage ou du corps y compris le cuir chevelu; dans une composition capillaire ; dans une composition de protection solaire, de bronzage artificiel ou de soin après-soleil.

10. Utilisation selon l'une des revendications précédentes, pour le traitement cosmétique de la peau, des muqueuses et/ou des fibres kératiniques, notamment pour le traitement cosmétique et/ou le soin cosmétique des peaux, des cheveux et/ou des ongles abîmés et/ou âgés.

11. Composition cosmétique ou pharmaceutique destinée à améliorer l'hydratation de la peau, des muqueuses et/ou des fibres kératiniques, comprenant au moins un dérivé de formule (I) tel que défini à l'une des revendications 1 à 6.

12. Composition selon la revendication 11, dans laquelle les dérivés de polyaminoacide sont utilisés, seuls ou en mélange, en une quantité de 0,001 à 30% en poids par rapport au poids total de la composition, de préférence en une quantité de 0,01 à 15% en poids par rapport au poids total de la composition.

13. Composition selon l'une des revendications 11 à 12, se présentant :

- sous la forme d'un produit de maquillage de la peau du visage, du corps ou des lèvres ou des fibres kératiniques (ongles, cils, sourcils, cheveux), tel qu'un fond de teint, une crème teintée, un fard à joues ou à paupières, une poudre libre ou compacte, un stick anti-cernes, un stick camouflant, un mascara, un rouge à lèvres, un vernis-à-ongles,
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil;
- sous la forme d'une composition capillaire, et notamment une crème ou un gel de soin des cheveux, notamment un produit hydratant,
- d'une composition de protection solaire, de bronzage artificiel (autobronzant) ou de soin après-soleil.

Office européen des brevets

# RAPPORT DE RECHERCHE EUROPEENNE

Numéro de la demande
EP 02 29 0453

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | EP 0 928 608 A (AJINOMOTO KK)<br>14 juillet 1999 (1999-07-14)<br>* page 5, ligne 2 - page 5, ligne 11 *<br>* page 3, ligne 39 - ligne 44; revendication 1 *<br>--- | 1,2,11 | A61K7/06<br>A61K7/48 |
| X | DATABASE WPI<br>Section Ch, Week 200020<br>Derwent Publications Ltd., London, GB;<br>Class D21, AN 2000-232901<br>XP002188278<br>& JP 2000 053524 A (AJINOMOTO KK),<br>22 février 2000 (2000-02-22)<br>* abrégé *<br>--- | 1,2,11 | |
| A | MORELLE J: "LIPOAMINOACIDES ET COSMETOLOGIE"<br>PARFUMS COSMETIQUES SAVONS DE FRANCE, PARIS, FR,<br>vol. 3, no. 2,<br>1 février 1973 (1973-02-01), pages 82-93,<br>XP000106572<br>* page 92, colonne de droite, ligne 13 - ligne 16 *<br>--- | 1,2 | **DOMAINES TECHNIQUES RECHERCHES (Int.Cl.7)**<br>A61K |
| D,A | PATENT ABSTRACTS OF JAPAN<br>vol. 016, no. 528 (C-1001),<br>29 octobre 1992 (1992-10-29)<br>& JP 04 198114 A (AJINOMOTO CO INC),<br>17 juillet 1992 (1992-07-17)<br>* abrégé *<br>--- | 1,2,11 | |
| A | PATENT ABSTRACTS OF JAPAN<br>vol. 013, no. 260 (C-607),<br>15 juin 1989 (1989-06-15)<br>& JP 01 061412 A (SEIWA KASEI:KK),<br>8 mars 1989 (1989-03-08)<br>* abrégé *<br>---<br>-/-- | 1,2,11 | |

Le présent rapport a été établi pour toutes les revendications

| Lieu de la recherche | Date d'achèvement de la recherche | Examinateur |
|---|---|---|
| LA HAYE | 18 juin 2002 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

Numéro de la demande
EP 02 29 0453

# RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | GALLOT B ET AL: "LIQUID CRYSTALLINE PHASES AND EMULSIFYING PROPERTIES OF BLOCK COPOLYMER HYDROPHOBIC ALIPHATIC AND HYDROPHILIC PEPTIDIC CHAINS" ACS SYMPOSIUM SERIES, WASHINGTON, DC, US, no. 384, 1989, pages 116-128, XP000989464 ISSN: 0097-6156 * page 116 - page 117, ligne 20 * | 11 | |
| D,A | FR 2 776 510 A (OREAL) 1 octobre 1999 (1999-10-01) * revendications 1,24 * | 1,2,11 | |
| D,A | FR 2 533 209 A (CENTRE NAT RECH SCIENT) 23 mars 1984 (1984-03-23) * page 9, ligne 15 - ligne 17; revendications 1,6,8,9 * | 1,2,11 | |
| A | US 4 147 782 A (FOXX MARY ELIZABETH ET AL) 3 avril 1979 (1979-04-03) * colonne 1, ligne 47 - ligne 62; revendication 1 * | 11 | DOMAINES TECHNIQUES RECHERCHES (Int.Cl.7) |
| A | PATENT ABSTRACTS OF JAPAN vol. 1995, no. 11, 26 décembre 1995 (1995-12-26) & JP 07 223920 A (KAO CORP), 22 août 1995 (1995-08-22) * abrégé * | 11 | |

Le présent rapport a été établi pour toutes les revendications

| Lieu de la recherche | Date d'achèvement de la recherche | Examinateur |
|---|---|---|
| LA HAYE | 18 juin 2002 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 02 29 0453

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-06-2002

| Document brevet cité au rapport de recherche | | Date de publication | Membre(s) de la famille de brevet(s) | | Date de publication |
|---|---|---|---|---|---|
| EP 0928608 | A | 14-07-1999 | JP | 11246841 A | 14-09-1999 |
| | | | JP | 11240828 A | 07-09-1999 |
| | | | EP | 0928608 A2 | 14-07-1999 |
| | | | JP | 2000007942 A | 11-01-2000 |
| JP 2000053524 | A | 22-02-2000 | AUCUN | | |
| JP 04198114 | A | 17-07-1992 | JP | 2890834 B2 | 17-05-1999 |
| JP 01061412 | A | 08-03-1989 | JP | 2564561 B2 | 18-12-1996 |
| FR 2776510 | A | 01-10-1999 | FR | 2776510 A1 | 01-10-1999 |
| | | | AU | 2284099 A | 18-10-1999 |
| | | | CA | 2324302 A1 | 07-10-1999 |
| | | | EP | 1066016 A1 | 10-01-2001 |
| | | | WO | 9949837 A1 | 07-10-1999 |
| | | | JP | 2002509869 T | 02-04-2002 |
| FR 2533209 | A | 23-03-1984 | FR | 2533209 A1 | 23-03-1984 |
| | | | DE | 3363079 D1 | 22-05-1986 |
| | | | EP | 0105777 A1 | 18-04-1984 |
| | | | JP | 59078149 A | 04-05-1984 |
| | | | US | 4600526 A | 15-07-1986 |
| US 4147782 | A | 03-04-1979 | GB | 2041963 A ,B | 17-09-1980 |
| | | | AU | 522041 B2 | 13-05-1982 |
| | | | AU | 4439579 A | 18-06-1981 |
| | | | BE | 874196 A1 | 29-05-1979 |
| | | | DE | 2906213 A1 | 04-09-1980 |
| | | | FR | 2450107 A1 | 26-09-1980 |
| | | | JP | 55116797 A | 08-09-1980 |
| | | | NL | 7902550 A ,B, | 06-10-1980 |
| JP 07223920 | A | 22-08-1995 | AUCUN | | |

EPO FORM P0460

Pour tout renseignement concernant cette annexe : voir Journal Officiel de l'Office européen des brevets, No.12/82